# EUROPEAN PATENT SPECIFICATION

(11) **EP 3 685 871 B1**
(45) Date of publication and mention of the grant of the patent: **29.09.2021**
(21) Application number: 19153029.4
(22) Date of filing: 22.01.2019
(51) Int. Cl.: A61M 25/00, A61M 3/02

(54) **A TEMPERATURE SENSING LAVAGE CATHETER AND METHOD OF MANUFATCURING THEREOF**
TEMPERATURERFASSENDER LAVAGE-KATHETER UND HERSTELLUNGSVERFAHREN DES KATHETERS
CATHÉTER DE LAVAGE À DÉTECTION DE TEMPÉRATURE ET PROCÉDÉ DE FABRICATION DUDIT CATHÉTER

(43) Date of publication of application: 29.07.2020
(73) Proprietor: Eight Medical International B.V., 3045 AX Rotterdam (NL)
(72) Inventor: van der Werf, Eelko, 2811AG Reeuwijk (NL)
(74) Representative: IP HILLS NV

(56) References cited:
- WO-A1-2016/008725
- WO-A2-2007/048068
- WO-A2-2013/016437
- US-A1- 2010 204 765
- US-A1- 2018 050 172

## Description

### Field of the Invention

The present invention generally relates to the measurement of the temperature of a fluid in a body cavity of a patient with the use of a catheter comprising a temperature sensor. More particularly, the present invention generally relates to the measurement of the temperature of a fluid in a body cavity of a patient during therapeutic irrigation of the body cavity with the fluid with a lavage catheter comprising a temperature sensor. The present invention also relates to a method for manufacturing such a catheter.

### Background of the Invention

During therapeutic irrigation, also referred to as lavage, a patient's body cavity is typically infused with one or more fluids or irrigants, or one or more solutions for therapy or diagnosis. For example, a patient's body cavity may be infused with therapeutic fluids or nutritive agents, or a patient's body cavity may be infused with chemotherapeutic fluids during treatment of a cancer. This way, a patient's body cavity and more particularly the internal surface of the body cavity may be washed by flowing the cavity with for example water or an irrigant for therapy or diagnosis.

Examples of therapeutic irrigation are an antiseptic lavage, a bronchoalveolar lavage, a gastric lavage, a peritoneal lavage, an arthroscopic lavage, a ductal lavage, a nasal irrigation, an ear lavage, etc.. A bronchoalveolar lavage is for example a medical procedure in which a bronchoscope is passed through the mouth or nose of a patient into the lungs and where fluid is squirted into a small part of the lung and then collected for examination. It is typically performed to diagnose lung disease. Bronchoalveolar lavage is commonly used to diagnose infections in for example people with immune system problems, pneumonia in people on ventilators, some types of lung cancer, and scarring of the lung. It is the most common method used to sample the epithelial lining fluid and to determine the protein composition of the pulmonary airways. It is often used in immunological research as a means of sampling cells or pathogen levels in the lung. Gastric lavage, also commonly called stomach pumping or gastric irrigation, is the process of cleaning out the contents of the stomach. Gastric lavage involves the passage of a tube, such as for example an Ewald tube, via the mouth or nose down into the stomach followed by sequential administration and removal of small volumes of liquid. Small amounts of warm water or saline are administered and, via a siphoning action, removed again. Diagnostic peritoneal lavage, also referred to as DPL, or diagnostic peritoneal aspiration, also referred to as DPA, is a surgical diagnostic procedure to determine if there is free floating fluid such as blood in the abdominal cavity. A catheter is inserted towards the pelvis and aspiration of material attempted using a syringe. If no blood is aspirated, saline is infused and then drained to be sent for analysis. Ductal lavage is a method used to collect cells from milk ducts in the breast. A catheter is inserted into the nipple, and a small amount of salt water is released into the duct. The water picks up breast cells and is removed. Ductal lavage may be used in addition to clinical breast examination and mammography to detect breast cancer. A pulsed lavage is yet another example of therapeutic irrigation and it consists in delivering an irrigant, usually normal saline, under direct pressure that is produced by an electrically powered device and is useful in cleaning e.g. chronic wounds.

The one or more fluids or the one or more solutions used for therapeutic irrigation may originate from a liquid-filled bag or container and a fluid delivery line may deliver the fluids under gravity and/or applied pressure from the container to the patient's body cavity. In most of the examples above, a catheter is introduced in the body cavity to deliver the one or more fluids or solutions to the body cavity. The fluid may be removed from the body cavity after the body cavity has been washed by flowing the cavity with the fluid, for example when the fluid has flown the body cavity enough according to the requirements for therapy and/or when the fluid has flown the body cavity enough for diagnosis purposes. The direct delivery of fluids to a patient's body cavity allows for higher doses of treatment, and thereby increases the efficiency of the treatment. Additionally, the local concentration of fluids within a patient's body cavity minimizes the rest of the patient's body exposure to the treatment, thereby reducing some side effects of the treatment. In the meantime, heating the solution may also improve the absorption of fluid by cells of the body cavity.

A catheter is a thin tube made from medical grade materials that can be inserted into the body to treat diseases or perform a surgical procedure. Catheters can be inserted into a body cavity, duct, or vessel. Functionally, they allow drainage, administration of fluids or gases, access by surgical instruments, and perform a wide variety of other tasks depending on the type of catheter. The process of inserting a catheter is "catheterization". In most uses, a catheter is a thin, flexible tube although catheters are available in varying levels of stiffness depending on the application. A catheter left inside the body, either temporarily or permanently, may be referred to as an indwelling catheter, for example, a peripherally inserted central catheter. A permanently inserted catheter may be referred to as a "permcath".

When flowing a body cavity of a patient with a fluid such as done - among others - in the context of therapeutic irrigation or lavage, it is important to maintain the temperature of the fluid in the fluid delivery lines within a desirable and safe temperature range upon entering the patient so as to eliminate any potential risk of thermal shock and injury induced by the fluids to the patient. It is therefore very important to accurately monitor the temperature of the fluid as close as possible to the body cavity such that the temperature of the fluid is within a desirable and safe temperature range to allow for example treatment and/or diagnosis of the body cavity, while eliminating any potential risk of thermal shock and injury induced by the fluid to the patient and more particularly to the body cavity. If the fluid is cooled or heated too much, there exists a risk of degradation of the nature and of the stability of the fluid delivered to the patient, which could endanger his health and/or jeopardize the efficiency of the treatment. A very precise control of the temperature of the fluid in the body cavity enables an appropriate treatment of the body cavity and/or an appropriate collection of for example cells of the body cavity for diagnosis purposes.

Several examples of catheter comprising one or more temperature sensors exist. However, there is a risk that a temperature sensor fails. When a temperature sensor fails, the failed catheter may have to be replaced. This compounds patient discomfort and it also increases the risk of injury and infection for the patient when the failed catheter is removed from the patient's body and when a new catheter is introduced as replacement. A further risk resulting from possible failure of a temperature sensor is that the failure will not be detected and that other systems such as for example the fluid delivery system might depend on the erroneous temperature sensor reading. For example, the fluid delivery system may fail to properly operate when relying on a temperature reading provided by a failed temperature sensor and may delivery fluid at a too high or too low temperature, thereby risking thermal shock and injury induced by the fluid to the patient and more particularly to the body cavity.

The U.S. Patent Application US2002/0082587A1 entitled "Foley Catheter Having Redundant Temperature Sensors and Method" describes a Foley catheter with redundant temperature sensors which is introduced into the bladder of a patient to drain urine from the bladder. The Foley catheter of US2002/0082587A1 comprises a drainage lumen, an integrated temperature sensor lumen and a balloon inflation lumen. The integrated temperature sensor of US2002/0082587A1 comprises an integrated sensor element 35 visible on Fig. 2 of US2002/0082587A1 which is advanced via the drainage lumen to be disposed at the distal end of the catheter. When the integrated sensor element 35 of US2002/0082587A1 fails, the user of the catheter attaches an adapter to the catheter and an additional sensor with an additional temperature sensor 38 visible on Fig. 2 of US2002/0082587A1 advances through the drainage lumen of the catheter to position the temperature sensor 38 in the distal end of the catheter.

The Foley catheter described in US2002/0082587A1 requires the use of two temperature sensors 35;38 positioned at the distal end of the catheter to guarantee an accurate and reliable measurement of the temperature of the urine in the bladder of the patient. The distal end of the catheter must be designed to be able to simultaneously host both temperature sensors for a reliable measurement. The design of the catheter is therefore not compact, which compounds discomfort and possibly injury for the patient upon introduction of the catheter along the ureter or any other body cavity. Additionally, the temperature sensor wires of both temperature sensors are advanced towards the distal end of the catheter via the drainage lumen. In other words, the temperature wires are in direct contact with urine in the fluid passage in the drainage lumen when urine is being drained from the bladder by the catheter. As urine flows in the drainage lumen, the temperature sensors of US2002/0082587A1 are consequently contaminated by the direct contact with the urine. The patient could be endangered if the temperature sensor was not thoroughly cleaned before being introduced in the catheter, or if the temperature sensor was not properly electrically isolated from the fluid.
WO2013/016437 A2 describes a multi-lumen catheter for cooling of a brain or spinal column.
US 2010/204765 A1 describes a therapeutic hypothermia system infusing fluid into a patient via a catheter.

### Summary of the Invention

Unit conversion: 1 inch = 2,54 cm

The invention is defined in appended claims 1-15. It is an objective of the present invention to disclose a catheter for measuring temperature of a fluid in a body cavity which overcomes the above identified shortcomings of existing solutions. More particularly, it is an objective to disclose a catheter with an integrated temperature sensor which indirectly and accurately measures the temperature of a fluid in a body cavity.

According to a first aspect of the present invention, the above defined objectives are realized by a catheter for measuring temperature of a fluid in a body cavity, wherein the catheter comprises a catheter body comprising a proximal end and an opposite distal end comprising a tip; and wherein the catheter body further comprises:
- an inflow lumen extending from an irrigation opening in a sidewall of the tip to the proximal end;
- an outflow lumen extending from a drainage opening in the sidewall of the tip to the proximal end; and
- a temperature sensor lumen comprising a temperature sensor; wherein the temperature sensor lumen is arranged closer to the outflow lumen than to the inflow lumen in the catheter body; and
wherein the temperature sensor comprises a sensing element positioned in the temperature sensor lumen at the tip of the distal end; and wherein the sensing element is configured to measure a temperature of a fluid in a body cavity when the distal end of the catheter body is inserted in a body cavity.

The catheter according to the present invention is used to deliver a fluid for lavage purposes and/or for therapeutic purposes and/or for diagnosis purposes to a body cavity of a patient in the context of a localized treatment of a specific body cavity. The catheter according to the present invention directly delivers the fluid to the body cavity as the catheter is introduced directly into the body cavity. The efficiency of the fluid on the body cavity is therefore maximized: the fluid is only delivered in the body cavity. The precision of the treatment/diagnosis is also maximized. The impact of the treatment and/or diagnosis on the patient is also minimized as the delivery of the fluid is local and does not spread to the whole body of the patient. An inflow lumen is arranged in the catheter body to deliver a fluid to the body cavity when the catheter is introduced in the body cavity. An outflow lumen is arranged in the catheter body to drain a fluid from the body cavity when the catheter is introduced in the body cavity. The catheter according to the present invention allows an indirect and accurate measurement of the temperature of a fluid in a body cavity when the catheter is introduced in the body cavity. The catheter according to the present invention comprises an integrated temperature sensor, wherein the sensing element is positioned at the tip of the distal end of the catheter. When the distal end of the catheter according to the present invention is introduced into a body cavity, the sensing element is positioned as close as possible to the fluid in the body cavity. The sensing element is in direct contact with the tip of the catheter body. The outside surface of the tip of the catheter body is in direct contact with the fluid in the body cavity. The sensing element measures the temperature of the fluid through the tip of the catheter body. In other words, the temperature sensor according to the present invention indirectly measures the temperature of the fluid. The sensing element according to the present invention is arranged in the catheter body closer to the outflow lumen than to the inflow lumen. Preferably, the distance between the temperature sensor lumen in which the sensing element is positioned and the outflow lumen is twice the distance between the temperature sensor lumen and the inflow lumen. Alternatively, the distance between the temperature sensor lumen in which the sensing element is positioned and the outflow lumen is more than twice the distance between the temperature sensor lumen and the inflow lumen. By being closer to the outflow lumen than to the inflow lumen in the catheter body, the sensing element according to the present invention allows a more accurate and a more reliable measurement of the temperature of a fluid in the body cavity. The sensing element according to the present invention provides a user of the catheter with a more realistic feedback on the actual temperature of the fluid in the body cavity. The sensing element according to the present invention can for example indirectly measure the temperature of a fluid in a body cavity. The catheter according to the present invention can for example indirectly measure the temperature of a fluid in a body cavity with an accuracy comprised between +/-1°C and +/-0.1°C, preferably with an accuracy or precision comprised between or equal to +/-0.5°C and +/-0.1 °C.

According to the present invention, the catheter body comprises a temperature sensor lumen which is configured to host the temperature sensor. The temperature sensor lumen is formed in the catheter body. The temperature sensor lumen is independent from the inflow lumen, and the temperature sensor lumen is also independent from the outflow lumen. In other words, the temperature sensor lumen is neither in fluid communication with the inflow lumen nor in fluid communication with the outflow lumen. This way, the temperature sensor and the sensing element according to the present invention are never in direct contact with the fluid in the catheter body. The temperature sensor and the sensing element according to the present invention are therefore never contaminated by direct contact with a fluid in body cavity when measuring the temperature of the fluid. Additionally, the temperature sensor according to the present invention may be removed from the catheter, and because neither the temperature sensor nor the sensing element have ever been contaminated by direct contact with a fluid, the temperature sensor and the sensing element do not endanger the patient when the temperature of the fluid is measured.

The outflow lumen and the inflow lumen according to the present invention are independent from each other in the catheter body. In other words, the outflow lumen and the inflow lumen are arranged side by side in the catheter body of the catheter according to the present invention. In other words, the outflow lumen and the inflow lumen are arranged next to each other in the catheter body. Alternatively, the outflow lumen comprises the inflow lumen. In other words, the outflow lumen and the inflow lumen can alternatively be in fluid communication with each other when the inflow lumen is completely comprised in the outflow lumen. This way, the footprint of the catheter body is significantly reduced.

The distal end of the catheter body is designed to be easily introduced into the body cavity, without injuring the patient. For example, the tip of the catheter comprises silicon, such as for example clear silicone. Alternatively, the tip of the catheter according to the present invention comprises blue radiopaque silicone. The tip of the distal end of the catheter for example comprises clear 60 ShA silicone such as for example EL R402/60 and barium sulfate Blanc Fixe Grade HD 80. According to the present invention, the catheter body comprises silicone, such as for example silicone rubber. This way, the catheter body is heat resistant, low-temperature flexible and resistant to UV, ozone and radiation aging. Alternatively, the catheter body comprises polycarbonate. This way, the catheter is hygienic, easy and cheap to fabricate in series and in large quantities, and it can easily be disposed. Alternatively, the catheter body is made of thermoplastic. Alternatively, a catheter inflation valve comprises a polypropylene body. All materials used in the catheter according to the present invention are to meet ANSI/AAMI/ISO 10993-1:2003 requirements. The catheter body according to the present invention is cylindrical and has a diameter to conform to the French scale or French gauge system commonly used to measure the size of a catheter. The French size is three times the diameter in millimeters. For example, the French size of the catheter according to the present invention is 16. Alternatively, the French size of the catheter according to the present invention is 18. The French size is understood in the context of the present invention as a measure of the outer diameter of a catheter.

According to the present invention, the sensing element is for example a thermistor. More particularly, the sensing element according to the present invention is for example a coated thermistor used to minimize the risk of health hazard for the patient when the catheter is introduced into a body cavity. For example, the sensing element according to the present invention comprises a housing comprising polyvinyl chloride or PVC, such as for example a white medical grade PVC. The sensing element may further comprise a thermistor side port with comprises silicone such as a silicone rubber. This way, the sensing element is heat resistant, low-temperature flexible and resistant to UV, ozone and radiation aging. Alternatively, the temperature sensor is a thermocouple, a resistance thermometer, a silicon bandgap temperature sensor, a thermostatic switch, an infra-red temperature sensor, etc..

A body cavity is for example a natural body cavity, such as for example a bladder, a stomach, an abdomen, a lung, etc.. Alternatively, a body cavity in the context of the present invention is for example an artificial body cavity, such as for example the abdomen of a patient closed by a surgeon during surgery, or an artefact enclosing a part or the entirety of an organ of a patient in which the circulation of a fluid delivered by the catheter would occur.

A fluid according to the present invention is a highly concentrated, heated fluid directly delivered to a body cavity of a patient during lavage or therapeutic irrigation in the context of a treatment or a diagnosis. For example, a fluid according to the present invention may not comprise chemotherapeutic agents. Alternatively, a fluid according to the present invention is any suitable irrigant and/or solution which may be delivered to a body cavity by a catheter. For example, a fluid according to the present invention is a normal saline solution. Alternatively, a fluid according to the present invention is a highly concentrated, heated therapeutic fluid directly delivered to the bladder of the patient before, immediately after of after Transurethral Resection of Bladder Tumors surgery, also referred to as TURBT surgery. Alternatively, a fluid according to the present invention is a highly concentrated, heated therapeutic fluid directly delivered to the bladder of the patient before, immediately after of after a hyperthermic intra-vesical chemotherapy treatment, also referred to as HIVEC. Alternatively, a fluid according to the present invention is any fluid which is suitable and compatible with a hyperthermic treatment, or any therapeutic fluid and/or nutritive agents and/or chemotherapeutic fluids and/or immunotherapy fluids delivered during a treatment or a diagnosis. The fluid according to the present invention has a temperature comprised between 37 and 45°C, more particularly comprised between 39.5 and 40.5°C, or more particularly comprised between 41.8 and 42°C, or more particularly comprised between 43 and 44°C when it is in the body cavity. Delivery of the fluid to the body cavity can for example last several minutes, preferably one hour, or several hours.

According to the present invention, the inner diameter of the outflow lumen is larger than the inner diameter of the inflow lumen. More particularly, a ratio of the inner diameter of the inflow lumen over the inner diameter of the outflow lumen is comprised between 0.6 and 1. This way, obstructions in the outflow lumen due to body tissues being drained from the body cavity are prevented. Additionally, potential pressure issues in the fluid circulation system formed by the catheter are prevented thanks to a larger inner diameter of the outflow lumen than the inner diameter of the inflow lumen. This way, fluid that has already flown in the body cavity does not flow back into the inflow lumen. The risk of contamination of the inflow lumen by fluid that has been in contact with tissue is therefore lowered. The catheter according to the present invention is for example designed to meet or exceed a flow rate per A.S.T.M. F623-99 method for Foley catheters.

According to an optional aspect of the invention, the sensing element is positioned closer to the drainage opening than to the irrigation opening in the catheter body.

This way, the sensing element provides a more accurate temperature measurement of the temperature of the fluid in the body cavity by also measuring the temperature of the fluid when the fluid exists the body cavity. Measuring the temperature of the fluid in the body cavity and the fluid being drained from the body cavity provides a more realistic measurement of the actual temperature of the fluid in the body cavity.

According to an optional aspect of the invention, the temperature sensor lumen is arranged in the catheter body at the inside periphery of the catheter body.

This way, the sensing element is arranged in the temperature sensor lumen at the tip of the distal end of the catheter body and close to the outer surface of the catheter body in the thickness of the wall of the catheter body.

According to an optional aspect of the invention, a thickness of the sidewall of said tip is minimized at the distal end where the sensing element is positioned.

This way, the accuracy of the measurement of the sensing element is maximized and the response time of the sensing element is minimized.

According to an optional aspect of the invention, the temperature sensor lumen is separate in the catheter body from the inflow lumen and separate from the outflow lumen.

This way, the temperature sensor and the sensing element according to the present invention are never in direct contact with the fluid in the catheter body. The temperature sensor and the sensing element according to the present invention are therefore never contaminated by direct contact with a fluid in body cavity when measuring the temperature of the fluid. Additionally, the temperature sensor according to the present invention may be removed from the catheter, and because neither the temperature sensor nor the sensing element have ever been contaminated by direct contact with a fluid, the temperature sensor and the sensing element never endanger the patient's health.

According to an optional aspect of the invention, the catheter is a lavage catheter.

This way, the fluid can inflow in the body cavity via the irrigation opening and the fluid after having flown in the body cavity can flow out of the body cavity via the drainage opening. This way, there is a circulation of fluid in the body cavity, which allows for the therapeutic irrigation or lavage.

According to an optional aspect of the invention, the inflow lumen and the outflow lumen are configured to be coupled at the proximal end of the catheter body to a fluid circulation system; and the distal end of the catheter body is configured to be inserted into the body cavity.

For example, the proximal end of the catheter body comprises one or more valves which allow the connection of the proximal end of the catheter to for example devices and systems. For example, the proximal end of the catheter body comprises a valve to which a fluid circulation system is connected. This way, fluid is delivered from the fluid circulation system by the inflow lumen to the body cavity via the irrigation opening and fluid is drained towards the fluid circulation system by the outflow lumen via the drainage opening, thereby creating a fluid circulation in the body cavity. For example, the temperature sensor according to the present invention is introduced in the catheter body through one of the valve and is advanced in the temperature sensor lumen along a longitudinal direction of the catheter body until the sensing element is positioned at the tip of the distal end of the catheter body.

According to an optional aspect of the invention, the inflow lumen is further configured to allow a fluid of the fluid circulation system to flow from the proximal end to the body cavity through the irrigation opening; and wherein the outflow lumen is further configured to allow the fluid to flow out from the body cavity through the drainage opening to the proximal end.

According to an optional aspect of the invention, the catheter is a urinary catheter and the distal end is configured to be inserted into a bladder of a patient.

According to an optional aspect of the invention, the temperature sensor lumen extends in the catheter body from the proximal end to the distal end.

This way, the sensing element may be introduced at the proximal end of the catheter body and may then be advanced in the temperature sensor lumen towards the distal end of the catheter body until the sensing element reaches the tip of the distal end and remains in this position when measuring the temperature of a fluid flowing in the body cavity. Alternatively, the temperature sensor lumen extends from the distal end of the catheter body to a position along the catheter body which is not introduced in the body cavity when the catheter is under use. This way, the temperature sensor lumen remains available from the outside world to introduce the sensing element for measurement.

According to an optional aspect of the invention, the temperature sensor further comprises an electrical wire extending along the temperature sensor lumen between the sensing element and the proximal end of the catheter body.

This way, the data comprising information indicative for the temperature measurement performed by the sensing element is communicated to a remote system where the data can be analyzed and the temperature of the fluid in the body cavity can be determined. Alternatively, the sensing element transmits the temperature measurement wirelessly to a remote data analyzing system.

According to an optional aspect of the invention, the inflow lumen and the outflow lumen are not in fluid communication.

According to an optional aspect of the invention, a diameter of the outflow lumen is larger than a diameter of the inflow lumen.

According to an optional aspect of the invention, the catheter body further comprises an inflation balloon.

This way, when the catheter is introduced in the body cavity, the inflation balloon may be inflated to hold the catheter in the body cavity. When the inflation balloon is inflated, the diameter of the balloon is not to exceed four French sizes over the actual outer diameter of the catheter body, except for the 16FR which shall not exceed six French sizes over the actual outer diameter of the catheter body.

According to an optional aspect of the invention, the fluid is a therapeutic fluid.

According to a second aspect of the invention, there is provided a method for manufacturing a catheter for measuring temperature of a fluid in a body cavity, wherein the catheter comprises a catheter body comprising a proximal end and an opposite distal end comprising a tip; wherein the method comprises the steps of:
- providing a catheter for measuring temperature of a fluid in a body cavity, wherein the catheter comprises a catheter body comprising a proximal end and an opposite distal end comprising a tip;
- forming an irrigation opening in a sidewall of the tip;
- providing an inflow lumen extending from the irrigation opening to the proximal end;
- forming a drainage opening in the sidewall of the tip;
- providing an outflow lumen extending from the drainage opening to the proximal end;
- providing a temperature sensor lumen comprising a temperature sensor, wherein the temperature sensor comprises a sensing element;
- arranging the temperature sensor lumen closer to the outflow lumen than to the inflow lumen in the catheter body; and
- positioning the sensing element in the temperature sensor lumen at the tip of the distal end such that the sensing element measures a temperature of a fluid in a body cavity when the distal end of the catheter body is inserted into a body cavity.

The catheter according to the present invention is used to deliver a fluid for lavage purposes and/or for therapeutic purposes and/or for diagnosis purposes to a body cavity of a patient in the context of a localized treatment of a specific body cavity. The catheter according to the present invention directly delivers the fluid to the body cavity as the catheter is introduced directly into the body cavity. The efficiency of the fluid on the body cavity is therefore maximized: the fluid is only delivered in the body cavity. The precision of the treatment/diagnosis is also maximized. The impact of the treatment and/or diagnosis on the patient is also minimized as the delivery of the fluid is local and does not spread to the whole body of the patient. An inflow lumen is arranged in the catheter body to deliver a fluid to the body cavity when the catheter is introduced in the body cavity. An outflow lumen is arranged in the catheter body to drain a fluid from the body cavity when the catheter is introduced in the body cavity. The catheter according to the present invention allows an indirect and accurate measurement of the temperature of a fluid in a body cavity when the catheter is introduced in the body cavity. The catheter according to the present invention comprises an integrated temperature sensor, wherein the sensing element is positioned at the tip of the distal end of the catheter. When the distal end of the catheter according to the present invention is introduced into a body cavity, the sensing element is positioned as close as possible to the fluid in the body cavity. The sensing element is in direct contact with the tip of the catheter body. The outside surface of the tip of the catheter body is in direct contact with the fluid in the body cavity. The sensing element measures the temperature of the fluid through the tip of the catheter body. In other words, the temperature sensor according to the present invention indirectly measures the temperature of the fluid. The sensing element according to the present invention is arranged in the catheter body closer to the outflow lumen than to the inflow lumen. By being closer to the outflow lumen than to the inflow lumen in the catheter body, the sensing element according to the present invention allows a more accurate and a more reliable measurement of the temperature of a fluid in the body cavity. The sensing element according to the present invention provides a user of the catheter with a more realistic feedback on the actual temperature of the fluid in the body cavity. The sensing element according to the present invention can for example indirectly measure the temperature of a fluid in a body cavity. The catheter according to the present invention can for example indirectly measure the temperature of a fluid in a body cavity with an accuracy comprised between +/-1°C and +/-0.1°C, preferably with an accuracy or precision comprised between or equal to +/-0.5°C and +/-0.1°C.

According to the present invention, the catheter body comprises a temperature sensor lumen which is configured to host the temperature sensor. The temperature sensor lumen is formed in the catheter body. The temperature sensor lumen is independent from the inflow lumen, and the temperature sensor lumen is also independent from the outflow lumen. In other words, the temperature sensor lumen is neither in fluid communication with the inflow lumen nor in fluid communication with the outflow lumen. This way, the temperature sensor and the sensing element according to the present invention are never in direct contact with the fluid in the catheter body. The temperature sensor and the sensing element according to the present invention are therefore never contaminated by direct contact with a fluid in body cavity when measuring the temperature of the fluid. Additionally, the temperature sensor according to the present invention may be removed from the catheter, and because neither the temperature sensor nor the sensing element have ever been contaminated by direct contact with a fluid, the temperature sensor and the sensing element never endanger the patient's health.

The outflow lumen and the inflow lumen according to the present invention are independent from each other in the catheter body. In other words, the outflow lumen and the inflow lumen are arranged side by side in the catheter body of the catheter according to the present invention. In other words, the outflow lumen and the inflow lumen are arranged next to each other in the catheter body. Alternatively, the outflow lumen comprises the inflow lumen. In other words, the outflow lumen and the inflow lumen can alternatively be in fluid communication with each other when the inflow lumen is completely comprised in the outflow lumen. This way, the footprint of the catheter body is significantly reduced.

The distal end of the catheter body is designed to be easily introduced into the body cavity, without injuring the patient. For example, the tip of the catheter comprises silicon, such as for example clear silicone. Alternatively, the tip of the catheter according to the present invention comprises blue radiopaque silicone. The tip of the distal end of the catheter for example comprises clear 60 ShA silicone such as for example EL R402/60 and barium sulfate Blanc Fixe Grade HD 80. According to the present invention, the catheter body comprises silicone, such as for example silicone rubber. This way, the catheter body is heat resistant, low-temperature flexible and resistant to UV, ozone and radiation aging. Alternatively, the catheter body comprises polycarbonate. This way, the catheter is hygienic, easy and cheap to fabricate in series and in large quantities, and it can easily be disposed. Alternatively, the catheter body is made of thermoplastic. Alternatively, a catheter inflation valve comprises a polypropylene body. All materials used in the catheter according to the present invention are to meet ANSI/AAMI/ISO 10993-1:2003 requirements. The catheter body according to the present invention is cylindrical and has a diameter to conform to the French scale or French gauge system commonly used to measure the size of a catheter. The French size is three times the diameter in millimeters. For example, the French size of the catheter according to the present invention is 16. Alternatively, the French size of the catheter according to the present invention is 18. The French size is understood in the context of the present invention as a measure of the outer diameter of a catheter.

According to the present invention, the sensing element is for example a thermistor. More particularly, the sensing element according to the present invention is for example a coated thermistor used to minimize the risk of health hazard for the patient when the catheter is introduced into a body cavity. For example, the sensing element according to the present invention comprises a housing comprising polyvinyl chloride or PVC, such as for example a white medical grade PVC. The sensing element may further comprise a thermistor side port with comprises silicone such as a silicone rubber. This way, the sensing element is heat resistant, low-temperature flexible and resistant to UV, ozone and radiation aging. Alternatively, the temperature sensor is a thermocouple, a resistance thermometer, a silicon bandgap temperature sensor, a thermostatic switch, an infra-red temperature sensor, etc..

According to the present invention, a body cavity is for example a natural body cavity, such as for example a bladder, a stomach, an abdomen, a lung, etc.. Alternatively, a body cavity in the context of the present invention is for example an artificial body cavity, such as for example the abdomen of a patient closed by a surgeon during surgery, or an artefact enclosing a part or the entirety of an organ of a patient in which the circulation of a fluid delivered by the catheter would occur.

A fluid according to the present invention is a highly concentrated, heated fluid directly delivered to a body cavity of a patient during lavage or therapeutic irrigation in the context of a treatment or a diagnosis. For example, a fluid according to the present invention may not comprise chemotherapeutic agents. Alternatively, a fluid according to the present invention is any suitable irrigant and/or solution which may be delivered to a body cavity by a catheter. For example, a fluid according to the present invention is a normal saline solution. Alternatively, a fluid according to the present invention is a highly concentrated, heated therapeutic fluid directly delivered to the bladder of the patient before, immediately after of after Transurethral Resection of Bladder Tumors surgery, also referred to as TURBT surgery. Alternatively, a fluid according to the present invention is a highly concentrated, heated therapeutic fluid directly delivered to the bladder of the patient before, immediately after of after a hyperthermic intra-vesical chemotherapy treatment, also referred to as HIVEC. Alternatively, a fluid according to the present invention is any fluid which is suitable and compatible with a hyperthermic treatment, or any therapeutic fluid and/or nutritive agents and/or chemotherapeutic fluids and/or immunotherapy fluids delivered during a treatment or a diagnosis. The fluid according to the present invention has a temperature comprised between 37 and 45°C, more particularly comprised between 39.5 and 40.5°C, or more particularly comprised between 41.8 and 42°C, or more particularly comprised between 43 and 44°C when it is in the body cavity. Delivery of the fluid to the body cavity can for example last several minutes, preferably one hour, or several hours.

According to the present invention, the inner diameter of the outflow lumen is larger than the inner diameter of the inflow lumen. More particularly, a ratio of the inner diameter of the inflow lumen over the inner diameter of the outflow lumen is comprised between 0.6 and 1. This way, obstructions in the outflow lumen due to body tissues being drained from the body cavity are prevented. Additionally, potential pressure issues in the fluid circulation system formed by the catheter are prevented thanks to a larger inner diameter of the outflow lumen than the inner diameter of the inflow lumen. This way, fluid that has already flown in the body cavity does not flow back into the inflow lumen. The risk of contamination of the inflow lumen by fluid that has been in contact with tissue is therefore lowered. The catheter according to the present invention is for example designed to meet or exceed a flow rate per A.S.T.M. F623-99 method for Foley catheters.

### Brief Description of the Drawings

Fig. 1 schematically illustrates an embodiment of a catheter according to the present invention.
Fig. 2 schematically illustrates an embodiment of a catheter according to the present invention when the catheter is introduced into a body cavity of a patient.
Fig. 3 schematically illustrates an embodiment of a cross-section of the distal end of a catheter according to the present invention.
Fig. 4 schematically illustrates an embodiment of a cross-section of the catheter body according to the present invention.
Fig. 5 schematically illustrates an embodiment of the steps of the method according to the present invention.

### Detailed Description of Embodiment(s)

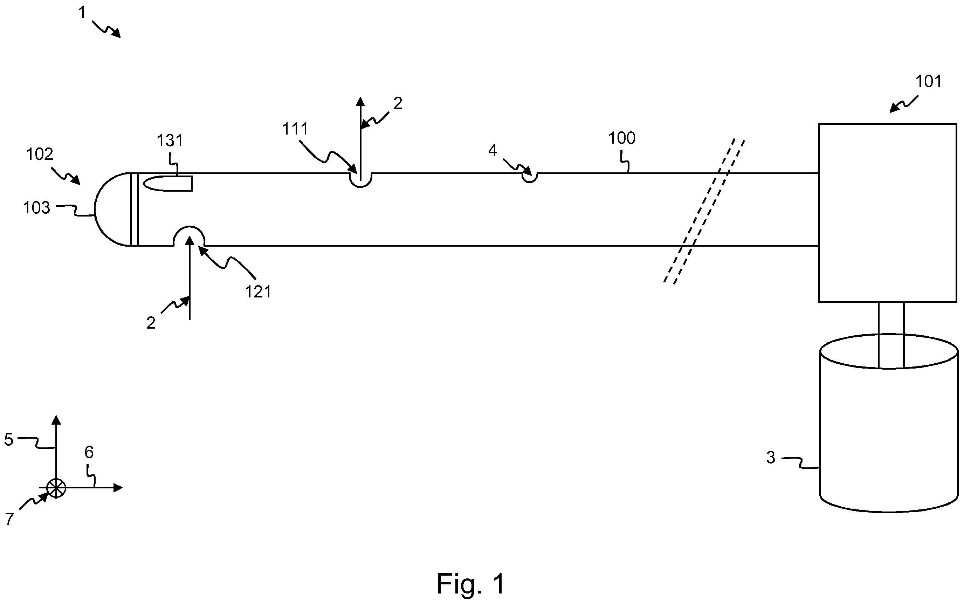

According to an embodiment shown in Fig. 1, a catheter 1 for measuring temperature of a fluid 2 in a body cavity comprises a catheter body 100. The catheter body 100 extends along a longitudinal direction 6. The traverse direction 5 and the direction 7 traverse to the longitudinal direction 6 and to the traverse direction 5 are also indicated on Fig. 1. The catheter body comprises a proximal end 101 and an opposite distal end 102. The distal end 102 comprises a tip 103. The catheter body 100 comprises an irrigation opening 111 from which fluid 2 is allowed flowing from the catheter body 100 to the body cavity. The catheter body 100 also comprises a drainage opening 121 from which fluid 2 is allowed flowing from the body cavity to the catheter body 100. A temperature sensor comprises a sensing element 131 positioned at the tip 103 of the distal end 102. The sensing element 131 measures a temperature of a fluid 2 in the body cavity when the distal end 102 of the catheter body 100 is introduced in a body cavity. Optionally, the catheter body 100 further comprises an inflation balloon 4. The sensing element 131 is positioned closer to the drainage opening 121 than to the irrigation opening 111 in the catheter body 100. A thickness of the sidewall of the tip 103 is minimized at the distal end 102 where the sensing element 131 is positioned. The catheter body 100 is coupled at its proximal end 101 to a fluid circulation system 3 and the distal end 102 is to be inserted into a body cavity 10. For example, the catheter 1 further comprises a funnel at the proximal end 101 which serves as a drain port for the outflow lumen, wherein the funnel is connected to the outflow lumen on one end and to the fluid circulation system 3 on an opposite end. Such a funnel for example comprises clear 60 ShA silicon, such as for example Elastosil LR 3003/60 A/B LSR. For example, the catheter body 100 of the catheter 1 comprises clear 70 ShA silicone, such as for example Silbione HCRA 4170 A/B HC. The catheter 1 for example further comprises an irrigation funnel at the proximal end 101 which serves as an irrigation port for the inflow lumen, wherein the irrigation funnel is connected to the inflow lumen on one end and to the fluid circulation system 3 on an opposite end. The catheter 1 for example further comprises an inflation valve at the proximal end 101, wherein the inflation valve is connected to the catheter body 100 on one end and to an inflation system for inflating the inflation balloon 4 on an opposite end. For example, the inflation valve is a Luer inflation valve comprising for example a polypropylene body. The inflation valve for example comprises an inflation valve collar which comprises Acrylonitrile Butadiene Styrene or ABS, such as for example LUSTRAN ABS348. A pigment of a first colour can be added to the LUSTRAN ABS348 for example for the 16FR catheter 1 and a pigment of second colour different from the first colour can be added to the LUSTRAN ABS348 for example for the 18FR catheter 1 such as the catheters 1 can be distinguished from each other. The inflation balloon 4 is for example a 20mL inflation balloon. According to an alternative embodiment, the inflation balloon may have a different volume suitable for the specific application.

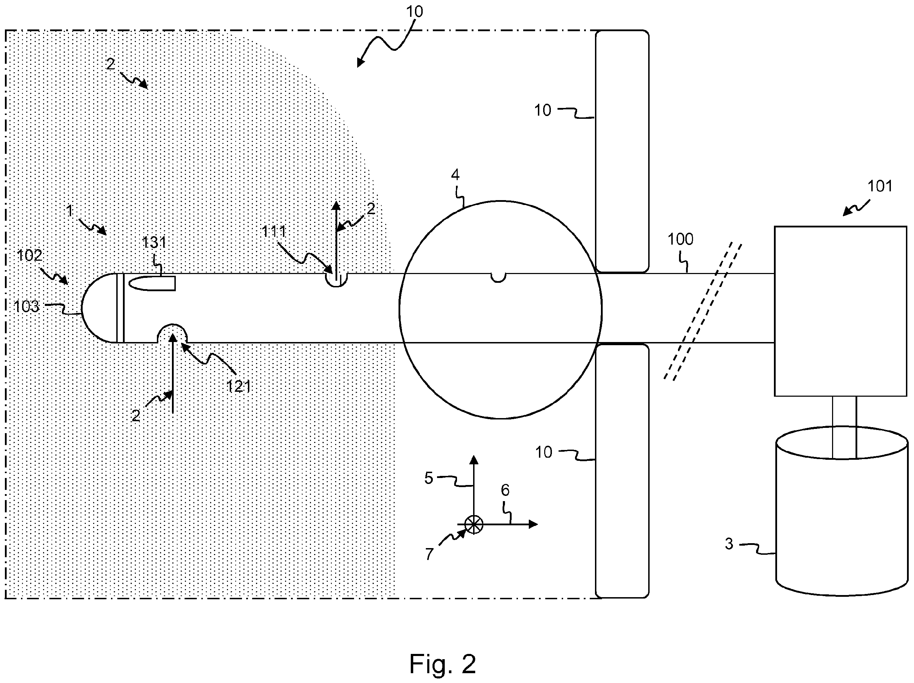

According to an embodiment shown in Fig. 2, a catheter 1 for measuring temperature of a fluid 2 in a body cavity 10 comprises a catheter body 100. The catheter body 100 extends along a longitudinal direction 6. The traverse direction 5 and the direction 7 traverse to the longitudinal direction 6 and to the traverse direction 5 are also indicated on Fig. 2. Components having identical reference numbers than on Fig. 1 perform the same function. The catheter body comprises a proximal end 101 and an opposite distal end 102. The distal end 102 comprises a tip 103. The catheter body 100 comprises an irrigation opening 111 from which fluid 2 is allowed flowing from the catheter body 100 to the body cavity 10. The catheter body 100 also comprises a drainage opening 121 from which fluid 2 is allowed flowing from the body cavity 10 to the catheter body 100. The body cavity is represented by the dash-dotted line on Figure 2. The fluid 2 is allowed to flow freely in the body cavity of Figure 2. A temperature sensor comprises a sensing element 131 positioned at the tip 103 of the distal end 102. The sensing element 131 measures a temperature of a fluid 2 in the body cavity 10 when the distal end 102 of the catheter body 100 is introduced in a body cavity 10. Optionally, the catheter body 100 further comprises an inflation balloon 4 which is inflated when the distal end 102 of the catheter body 100 is introduced into a body cavity 10. The sensing element 131 is positioned closer to the drainage opening 121 than to the irrigation opening 111 in the catheter body 100. A thickness of the sidewall of the tip 103 is minimized at the distal end 102 where the sensing element 131 is positioned. The catheter body 100 is coupled at its proximal end 101 to a fluid circulation system 3 and the distal end 102 is to be inserted into a body cavity 10.

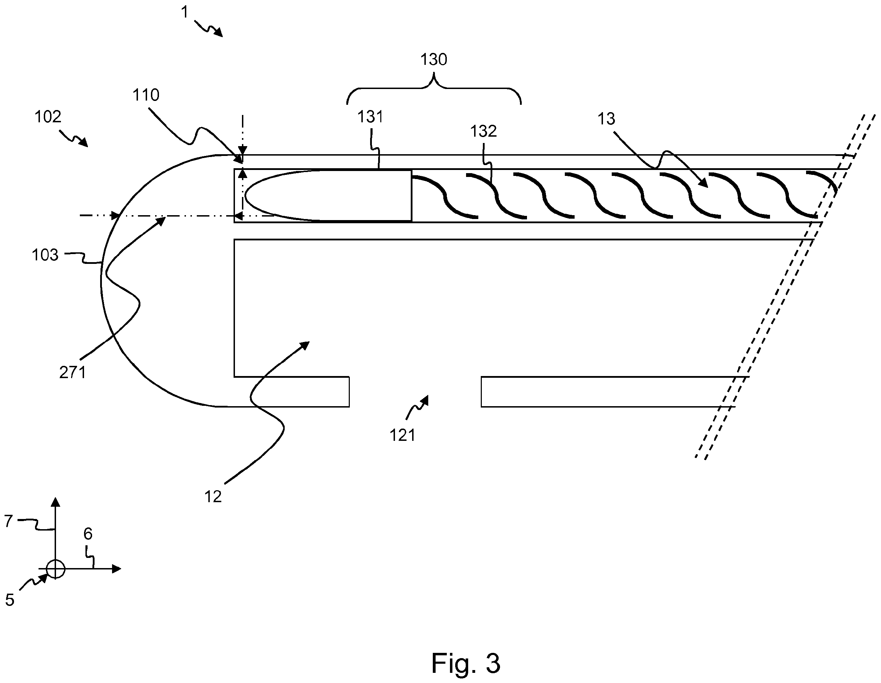

According to an embodiment shown in Fig. 3, a cross-section of the distal end 102 of a catheter body 100 of the catheter 1 is depicted. The catheter body 100 extends along a longitudinal direction 6. The traverse direction 5 and the direction 7 traverse to the longitudinal direction 6 and to the traverse direction 5 are also indicated on Fig. 3. The cross-section is defined along the longitudinal direction 6 of the catheter body 100 and along the direction 7 traverse to the longitudinal direction 6 and the traverse direction 5. Components having identical reference numbers than on Figs. 1 or 2 perform the same function. The distal end 102 comprises a tip 103. The catheter body 100 comprises an outflow lumen 12 and a temperature sensor lumen 13. The temperature sensor lumen 13 is arranged closed to the outflow lumen 12 than to the inflow lumen 11 in the catheter body 100. The outflow lumen 11 comprises a drainage opening 121 from which fluid 2 is allowed flowing from the body cavity 10 to the catheter body 100. The temperature sensor lumen 13 comprises a temperature sensor 130. The temperature sensor 130 comprises a sensing element 131 positioned at the tip 103 of the distal end 102. The sensing element 131 measures a temperature of a fluid in the body cavity when the distal end 102 of the catheter body 100 is introduced in a body cavity. The sensing element 131 is positioned closer to the drainage opening 121 than to the irrigation opening in the catheter body 100. A thickness of the sidewall 110 of the tip 103 along the direction 7 is minimized at the distal end 102 where the sensing element 131 is positioned. A thickness of the sidewall 271 of the tip 103 along the longitudinal direction 6 is minimized at the distal end 102 where the sensing element 131 is positioned. The temperature sensor lumen 13 is separate in the catheter body 100 from the outflow lumen 12 and also from the inflow lumen. The temperature sensor 130 further comprises an electrical wire 132 extending in the temperature sensor lumen 13 and along the longitudinal direction 6 of the catheter body 100. The electrical wire 132 extends preferably between the sensing element 131 and the proximal end of the catheter body 100.

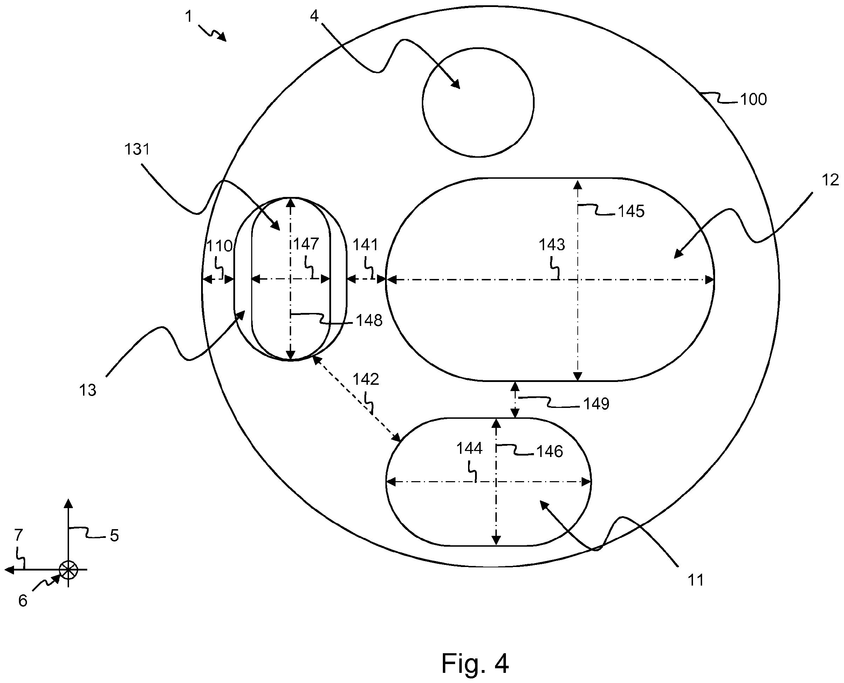

According to an embodiment shown in Fig. 4, a cross-section of the catheter body 100 of the catheter 1 is depicted. The catheter body 100 extends along a longitudinal direction 6. The traverse direction 5 and the direction 7 traverse to the longitudinal direction 6 and to the traverse direction 5 are also indicated on Fig. 4. The cross-section is defined along the direction 7 traverse to the longitudinal direction 6 and to the traverse direction 5 of the catheter body 100. Components having identical reference numbers than on Figs. 1 or 2 or 3 perform the same function. The catheter body 100 comprises an inflow lumen 11, an outflow lumen 12 and a temperature sensor lumen 13. Optionally, the catheter body 100 further comprises an inflation balloon 4 which is inflated when the distal end of the catheter 1 is introduced in a body cavity to keep the catheter 1 in the body cavity. The temperature sensor lumen 13 is arranged closer to the outflow lumen 12 than to the inflow lumen 11 in the catheter body 100. Preferably, the distance 141 between the temperature sensor lumen 13 in which the sensing element 131 is positioned and the outflow lumen 12 is twice the distance 142 between the temperature sensor lumen 13 and the inflow lumen 11. According to an alternative embodiment, the distance 141 between the temperature sensor lumen 13 in which the sensing element 131 is positioned and the outflow lumen 12 is more than twice the distance 142 between the temperature sensor lumen 13 and the inflow lumen 11, for example three, four, five, six, etc., times the distance 142 between the temperature sensor lumen 13 and the inflow lumen 11. A thickness of the sidewall 110 of the tip 103 is minimized at the distal end 102 where the sensing element 131 is positioned in the temperature sensor lumen 13. The inner diameter 143 of the outflow lumen 13 is larger than the inner diameter 144 of the inflow lumen 11. More particularly, a ratio of the inner diameter 144 of the inflow lumen 11 over the inner diameter 143 of the outflow lumen 13 is comprised between 0.6 and 1. This way, obstructions in the outflow lumen 13 due to body tissues being drained from the body cavity are prevented. The inner diameter 143 of the outflow lumen 13 is schematically depicted as extending along the direction 7 on Fig. 4. According to an alternative embodiment, the inner diameter 143 of the outflow lumen 13 could extend along any direction of the catheter body 100. The inner diameter 144 of the inflow lumen 11 is schematically depicted as extending along the direction 7 on Fig. 4. According to an alternative embodiment, the inner diameter 144 of the inflow lumen 11 could extend along any direction of the catheter body 100. For example, for a circular 16 French catheter having a diameter of 0.210 inches, the distance 141 between the temperature sensor lumen 13 and the outflow lumen 12 is for example 0.010 inches, which is the minimum internal wall thickness which can be manufactured; the distance 142 between the temperature sensor lumen 13 and the inflow lumen 11 is for example 0.025 inches; the distance 149 between the outflow lumen 12 and the inflow lumen 11 is for example 0.017 inches; the length 143 of the outflow lumen 12 along the direction 7 is for example 0.113 inches; the length 145 of the outflow lumen 12 along the traverse direction 5 is for example 0.064 inches; the length 144 of the inflow lumen 11 along the direction 7 is for example 0.084 inches; the length 146 of the inflow lumen 11 along the traverse direction 5 is for example 0.056 inches; the length 147 of the temperature sensor lumen 13 along the direction 7 is for example 0.047 inches; the length 148 of the temperature sensor lumen 13 along the traverse direction 5 is for example 0.070 inches; the inflation balloon 4 has for example a diameter of 0.033 inches; and the thickness of the sidewall 110 of the catheter 1 is, where the sensing element 131 is, for example 0.012 inches. For example, for a circular 18 French catheter having a diameter of 0.236 inches, the distance 141 between the temperature sensor lumen 13 and the outflow lumen 12 is for example 0.014 inches; the distance 142 between the temperature sensor lumen 13 and the inflow lumen 11 is for example 0.034 inches; the distance 149 between the outflow lumen 12 and the inflow lumen 11 is for example 0.018 inches; the length 143 of the outflow lumen 12 along the direction 7 is for example 0.132 inches; the length 145 of the outflow lumen 12 along the traverse direction 5 is for example 0.083 inches; the length 144 of the inflow lumen 11 along the direction 7 is for example 0.080 inches; the length 146 of the inflow lumen 11 along the traverse direction 5 is for example 0.051 inches; the length 147 of the temperature sensor lumen 13 along the direction 7 is for example 0.055 inches; the length 148 of the temperature sensor lumen 13 along the traverse direction 5 is for example 0.080 inches; the inflation balloon 4 has for example a diameter of 0.035 inches; and the thickness of the sidewall 110 of the catheter 1, where the sensing element 131 is, is for example 0.016 inches. According to an alternative embodiment, the inflow lumen 11 and the outflow lumen 12 have a circular cross-section along a plane comprising the longitudinal direction 6 and to the traverse direction 5 of the catheter body 100 and the diameter of the outflow lumen 12 defined along this cross-section is preferably larger than the diameter of the inflow lumen 11 defined along this cross-section. For example, the diameter of the outflow lumen 12 is 1.1 times larger than the diameter of the inflow lumen 11. According to alternative embodiments, the diameter of the outflow lumen 12 is for example 1.2 times larger than the diameter of the inflow lumen 11, or 1.3 times, or 1.4 times, or 1.5 times, or 1.6 times, etc..

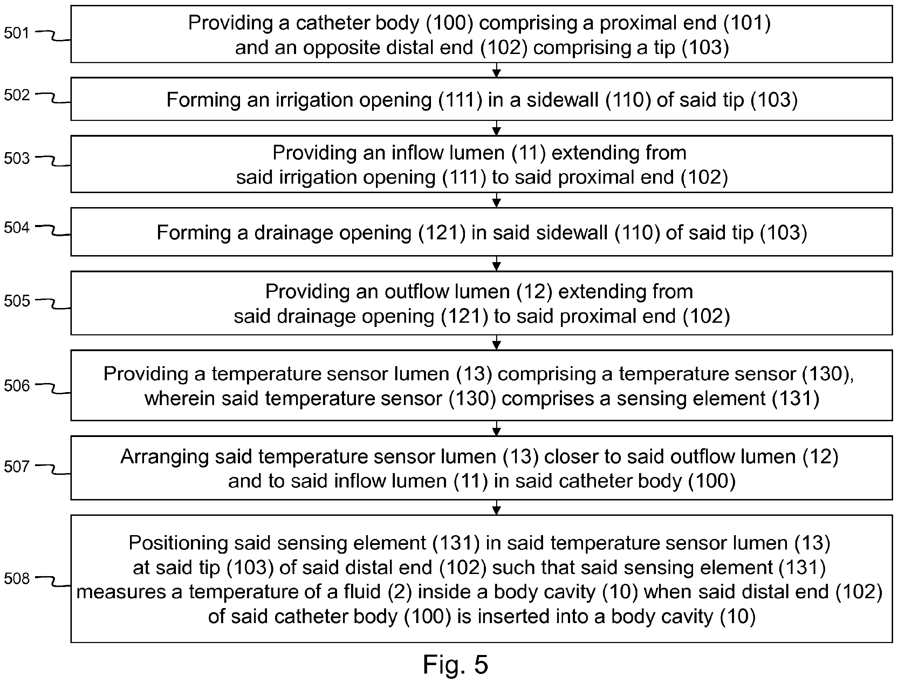

The steps of a method according to the present invention for manufacturing a catheter 1 for measuring a temperature of a fluid 2 in a body cavity 10 are depicted on Fig. 5. In step 501, the method comprises providing a catheter body 10 comprising a proximal end 101 and an opposite distal end 102 comprising a tip 103. In step 502, an irrigation opening 111 is then formed in the sidewall 110 of the tip 103. In step 503, an inflow lumen 11 is formed and is extended from the irrigation opening 111 to the proximal end 102. In step 504, a drainage opening 121 is then formed in the sidewall 110 of the tip 103. In step 505, an outflow lumen 12 is formed and is extending from the drainage opening 121 to the proximal end 102. In step 506, a temperature sensor lumen 13 is formed. The temperature sensor lumen 13 is provided with a temperature sensor 130, wherein the temperature sensor 130 comprises a sensing element 131. In step 507, the temperature sensor lumen 13 is arranged closer to the outflow lumen 12 than to the inflow lumen 11 in the catheter body 100. Finally, in step 508, the sensing element 131 is positioned in the temperature sensor lumen 13 at the tip 103 of the distal end 102 such that the sensing element 131measures a temperature of a fluid 2 inside a body cavity 10 when the distal end 102 of the catheter body 100 is inserted into a body cavity 10.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning of the claims are therefore intended to be embraced therein. In other words, it is contemplated to cover any and all modifications, variations that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A catheter (1) for measuring temperature of a fluid (2) in a body cavity (10), wherein said catheter (1) comprises a catheter body (100) comprising a proximal end (101) and an opposite distal end (102) comprising a tip (103); and wherein said catheter body (100) further comprises:
- an inflow lumen (11) extending from an irrigation opening (111) in a sidewall (110) of said tip (103) to said proximal end (101);
- an outflow lumen (12) extending from a drainage opening (121) in said sidewall (110) of said tip (103) to said proximal end (101); and
- a temperature sensor lumen (13) comprising a temperature sensor (130); wherein said temperature sensor lumen (13) is arranged closer to said outflow lumen (12) than to said inflow lumen (11) in said catheter body (100); and
wherein said temperature sensor (130) comprises a sensing element (131) positioned in said temperature sensor lumen (13) at said tip (103) of said distal end (102); and wherein said sensing element (131) is configured to measure a temperature of a fluid (2) in a body cavity (10) when said distal end (102) of said catheter body (100) is inserted into a body cavity (10).

2. A catheter (1) according to claim 1, wherein said sensing element (131) is positioned closer to said drainage opening (121) than to said irrigation opening (111) in said catheter body (100).

3. A catheter (1) according to any of the preceding claims, wherein said temperature sensor lumen (13) is arranged in said catheter body (100) at the outer periphery of said catheter body (100).

4. A catheter (1) according to any of the preceding claims, wherein a thickness of said sidewall (110) of said tip (103) is minimized at said distal end (102) where said sensing element (131) is positioned.

5. A catheter (1) according to any of the preceding claims, wherein said temperature sensor lumen (13) is separate in said catheter body (100) from said inflow lumen (11) and separate from said outflow lumen (12).

6. A catheter (1) according to any of the preceding claims, wherein said catheter (1) is a lavage catheter.

7. A catheter (1) according to any of the preceding claims, wherein said inflow lumen (11) and said outflow lumen (12) are configured to be coupled at said proximal end (101) of said catheter body (100) to a fluid circulation system (3); and wherein said distal end (102) of said catheter body (100) is configured to be inserted into said body cavity (10).

8. A catheter (1) according to claim 7, wherein said inflow lumen (11) is further configured to allow a fluid (2) of said fluid circulation system (3) to flow from said proximal end (101) to said body cavity (10) through said irrigation opening (111); and wherein said outflow lumen (12) is further configured to allow said fluid (2) to flow out from said body cavity (10) through said drainage opening (121) to said proximal end (101).

9. A catheter (1) according to any of the preceding claims, wherein said catheter (1) is a urinary catheter and wherein said distal end (102) is configured to be inserted into a bladder of a patient.

10. A catheter (1) according to any of the preceding claims, wherein said temperature sensor lumen (13) extends in said catheter body (100) from said proximal end (101) to said distal end (102).

11. A catheter (1) according to claim 10, wherein said temperature sensor (130) further comprises an electrical wire (132) extending along said temperature sensor lumen (13) between said sensing element (131) and said proximal end (101) of said catheter body (100).

12. A catheter (1) according to any of the preceding claims, wherein said inflow lumen (11) and said outflow lumen (12) are not in fluid communication.

13. A catheter (1) according to any of the preceding claims, wherein a diameter of said outflow lumen (12) is larger than a diameter of said inflow lumen (11).

14. A catheter (1) according to any of the preceding claims, wherein said catheter body (100) further comprises an inflation balloon (4).

15. A method for manufacturing a catheter (1) for measuring temperature of a fluid (2) in a body cavity (10), wherein said catheter (1) comprises a catheter body (100) comprising a proximal end (101) and an opposite distal end (102) comprising a tip (103); wherein said method comprises the steps of:
- providing a catheter body (100) comprising a proximal end (101) and an opposite distal end (102) comprising a tip (103);
- forming an irrigation opening (111) in a sidewall (110) of said tip (103);
- providing an inflow lumen (11) extending from said irrigation opening (111) to said proximal end (102);
- forming a drainage opening (121) in said sidewall (110) of said tip (103);
- providing an outflow lumen (12) extending from said drainage opening (121) to said proximal end (102);
- providing a temperature sensor lumen (13) comprising a temperature sensor (130), wherein said temperature sensor (130) comprises a sensing element (131);
- arranging said temperature sensor lumen (13) closer to said outflow lumen (12) than to said inflow lumen (11) in said catheter body (100); and
- positioning said sensing element (131) in said temperature sensor lumen (13) at said tip (103) of said distal end (102) such that said sensing element (131) measures a temperature of a fluid (2) in a body cavity (10) when said distal end (102) of said catheter body (100) is inserted into a body cavity (10).

## Patentansprüche

1. Katheter (1) zum Messen der Temperatur eines Fluids (2) in einer Körperhöhle (10), wobei der Katheter (1) einen Katheterkörper (100) umfasst, der ein proximales Ende (101) und ein gegenüberliegendes distales Ende (102) umfasst, das eine Spitze (103) umfasst; und wobei der Katheterkörper (100) ferner Folgendes umfasst:
- ein Einflusslumen (11), das sich von einer Spülöffnung (111) in einer Seitenwand (110) der Spitze (103) zu dem proximalen Ende (101) erstreckt;
- ein Ausflusslumen (12), das sich von einer Drainageöffnung (121) in der Seitenwand (110) der Spitze (103) zu dem proximalen Ende (101) erstreckt; und
- ein Temperatursensorlumen (13), das einen Temperatursensor (130) umfasst; wobei das Temperatursensorlumen (13) näher an dem Ausflusslumen (12) als an dem Einflusslumen (11) in dem Katheterkörper (100) angeordnet ist; und
wobei der Temperatursensor (130) ein Erfassungselement (131) umfasst, das in dem Temperatursensorlumen (13) an der Spitze (103) des distalen Endes (102) positioniert ist; und wobei das Erfassungselement (131) konfiguriert ist, um eine Temperatur eines Fluids (2) in einer Körperhöhle (10) zu messen, wenn das distale Ende (102) des Katheterkörpers (100) in eine Körperhöhle (10) eingeführt wird.

2. Katheter (1) nach Anspruch 1, wobei das Erfassungselement (131) näher an der Drainageöffnung (121) als an der Spülöffnung (111) in dem Katheterkörper (100) positioniert ist.

3. Katheter (1) nach einem der vorhergehenden Ansprüche, wobei das Temperatursensorlumen (13) in dem Katheterkörper (100) an dem Außenumfang des Katheterkörpers (100) angeordnet ist.

4. Katheter (1) nach einem der vorhergehenden Ansprüche, wobei eine Dicke der Seitenwand (110) der Spitze (103) an dem distalen Ende (102), an dem das Erfassungselement (131) positioniert ist, minimiert ist.

5. Katheter (1) nach einem der vorhergehenden Ansprüche, wobei das Temperatursensorlumen (13) in dem Katheterkörper (100) separat von dem Einflusslumen (11) und separat von dem Ausflusslumen (12) ist.

6. Katheter (1) nach einem der vorhergehenden Ansprüche, wobei der Katheter (1) ein Lavage-Katheter ist.

7. Katheter (1) nach einem der vorhergehenden Ansprüche, wobei das Einflusslumen (11) und das Ausflusslumen (12) konfiguriert sind, um an dem proximalen Ende (101) des Katheterkörpers (100) an ein Fluidzirkulationssystem (3) gekoppelt zu sein; und wobei das distale Ende (102) des Katheterkörpers (100) konfiguriert ist, um in die Körperhöhle (10) eingeführt zu werden.

8. Katheter (1) nach Anspruch 7, wobei das Einflusslumen (11) ferner konfiguriert ist, um zu ermöglichen, dass ein Fluid (2) des Fluidzirkulationssystems (3) von dem proximalen Ende (101) zu der Körperhöhle (10) durch die Spülöffnung (111) fließt; und wobei das Ausflusslumen (12) ferner konfiguriert ist, um zu ermöglichen, dass das Fluid (2) aus der Körperhöhle (10) durch die Drainageöffnung (121) zu dem proximalen Ende (101) fließt.

9. Katheter (1) nach einem der vorhergehenden Ansprüche, wobei der Katheter (1) ein Harnkatheter ist und wobei das distale Ende (102) konfiguriert ist, um in eine Blase eines Patienten eingeführt zu werden.

10. Katheter (1) nach einem der vorhergehenden Ansprüche, wobei sich das Temperatursensorlumen (13) in dem Katheterkörper (100) von dem proximalen Ende (101) zu dem distalen Ende (102) erstreckt.

11. Katheter (1) nach Anspruch 10, wobei der Temperatursensor (130) ferner einen elektrischen Draht (132) umfasst, der sich entlang des Temperatursensorlumens (13) zwischen dem Erfassungselement (131) und dem proximalen Ende (101) des Katheterkörpers (100) erstreckt.

12. Katheter (1) nach einem der vorhergehenden Ansprüche, wobei das Einflusslumen (11) und das Ausflusslumen (12) nicht in Fluidkommunikation sind.

13. Katheter (1) nach einem der vorhergehenden Ansprüche, wobei ein Durchmesser des Ausflusslumens (12) größer als ein Durchmesser des Einflusslumens (11) ist.

14. Katheter (1) nach einem der vorhergehenden Ansprüche, wobei der Katheterkörper (100) ferner einen Aufblasballon (4) umfasst.

15. Verfahren zur Herstellung eines Katheters (1) zum Messen der Temperatur eines Fluids (2) in einer Körperhöhle (10), wobei der Katheter (1) einen Katheterkörper (100) umfasst, der ein proximales Ende (101) und ein gegenüberliegendes distales Ende (102) umfasst, das eine Spitze (103) umfasst; wobei das Verfahren die folgenden Schritte umfasst:
- Bereitstellen eines Katheterkörpers (100), der ein proximales Ende (101) und ein gegenüberliegendes distales Ende (102) umfasst, das eine Spitze (103) umfasst;
- Bilden einer Spülöffnung (111) in einer Seitenwand (110) der Spitze (103);
- Bereitstellen eines Einflusslumens (11), das sich von der Spülöffnung (111) zu dem proximalen Ende (102) erstreckt;
- Bilden einer Drainageöffnung (121) in der Seitenwand (110) der Spitze (103);
- Bereitstellen eines Ausflusslumens (12), das sich von der Drainageöffnung (121) zu dem proximalen Ende (102) erstreckt;
- Bereitstellen eines Temperatursensorlumens (13), das einen Temperatursensor (130) umfasst, wobei der Temperatursensor (130) ein Erfassungselement (131) umfasst;
- Anordnen des Temperatursensorlumens (13) näher an dem Ausflusslumen (12) als an dem Einflusslumen (11) in dem Katheterkörper (100); und
- Positionieren des Erfassungselements (131) in dem Temperatursensorlumen (13) an der Spitze (103) des distalen Endes (102), sodass das Erfassungselement (131) eine Temperatur eines Fluids (2) in einer Körperhöhle (10) misst, wenn das distale Ende (102) des Katheterkörpers (100) in eine Körperhöhle (10) eingeführt wird.

## Revendications

1. Cathéter (1) destiné à mesurer la température d'un fluide (2) dans une cavité corporelle (10), ledit cathéter (1) comprenant un corps de cathéter (100) qui comprend une extrémité proximale (101) et une extrémité distale (102) opposée comprenant une pointe (103); et ledit corps de cathéter (100) comprend en outre :
- une lumière d'entrée (11) s'étendant d'une ouverture d'irrigation (111) dans une paroi latérale (110) de ladite pointe (103) à ladite extrémité proximale (101) ;
- une lumière de sortie (12) s'étendant d'une ouverture de drainage (121) dans ladite paroi latérale (110) de ladite pointe (103) à ladite extrémité proximale (101) ; et
- une lumière de capteur de température (13) comprenant un capteur de température (130) ; ladite lumière de capteur de température (13) étant agencée plus près de ladite lumière de sortie (12) que de ladite lumière d'entrée (11) dans ledit corps de cathéter (100) ; et
ledit capteur de température (130) comprenant un élément de détection (131) positionné dans ladite lumière de capteur de température (13) au niveau de ladite pointe (103) de ladite extrémité distale (102); et ledit élément de détection (131) étant conçu pour mesurer la température d'un fluide (2) dans une cavité corporelle (10) lorsque ladite extrémité distale (102) dudit corps de cathéter (100) est insérée dans une cavité corporelle (10).

2. Cathéter (1) selon la revendication 1, ledit élément de détection (131) étant positionné plus près de ladite ouverture de drainage (121) que de ladite ouverture d'irrigation (111) dans ledit corps de cathéter (100).

3. Cathéter (1) selon l'une quelconque des revendications précédentes, ladite lumière de capteur de température (13) étant agencée dans ledit corps de cathéter (100) à la périphérie externe dudit corps de cathéter (100).

4. Cathéter (1) selon l'une quelconque des revendications précédentes, l'épaisseur de ladite paroi latérale (110) de ladite pointe (103) étant minimisée au niveau de ladite extrémité distale (102) où ledit élément de détection (131) est positionné.

5. Cathéter (1) selon l'une quelconque des revendications précédentes, ladite lumière de capteur de température (13) étant séparée dans ledit corps de cathéter (100) de ladite lumière d'entrée (11) et séparée de ladite lumière de sortie (12).

6. Cathéter (1) selon l'une quelconque des revendications précédentes, ledit cathéter (1) étant un cathéter de lavage.

7. Cathéter (1) selon l'une quelconque des revendications précédentes, ladite lumière d'entrée (11) et ladite lumière de sortie (12) étant conçues pour être couplées au niveau de ladite extrémité proximale (101) dudit corps de cathéter (100) à un système de circulation de fluide (3) ; et ladite extrémité distale (102) dudit corps de cathéter (100) étant conçue pour être insérée dans ladite cavité corporelle (10).

8. Cathéter (1) selon la revendication 7, ladite lumière d'entrée (11) étant en outre conçue pour permettre à un fluide (2) dudit système de circulation de fluide (3) de s'écouler de ladite extrémité proximale (101) à ladite cavité corporelle (10) à travers ladite ouverture d'irrigation (111) ; et ladite lumière de sortie (12) étant en outre conçue pour permettre audit fluide (2) de s'écouler de ladite cavité corporelle (10) à travers ladite ouverture de drainage (121) jusqu'à ladite extrémité proximale (101).

9. Cathéter (1) selon l'une quelconque des revendications précédentes, ledit cathéter (1) étant un cathéter urinaire et ladite extrémité distale (102) étant conçue pour être insérée dans la vessie d'un patient.

10. Cathéter (1) selon l'une quelconque des revendications précédentes, ladite lumière de capteur de température (13) s'étendant dans ledit corps de cathéter (100) de ladite extrémité proximale (101) à ladite extrémité distale (102).

11. Cathéter (1) selon la revendication 10, ledit capteur de température (130) comprenant en outre un fil électrique (132) qui s'étend le long de ladite lumière de capteur de température (13) entre ledit élément de détection (131) et ladite extrémité proximale (101) dudit corps de cathéter (100).

12. Cathéter (1) selon l'une quelconque des revendications précédentes, ladite lumière d'entrée (11) et ladite lumière de sortie (12) n'étant pas en communication fluidique.

13. Cathéter (1) selon l'une quelconque des revendications précédentes, le diamètre de ladite lumière de sortie (12) étant plus grand que le diamètre de ladite lumière d'entrée (11).

14. Cathéter (1) selon l'une quelconque des revendications précédentes, ledit corps de cathéter (100) comprend en outre un ballonnet de gonflage (4).

15. Procédé de fabrication d'un cathéter (1) destiné à mesurer la température d'un fluide (2) dans une cavité corporelle (10), ledit cathéter (1) comprenant un corps de cathéter (100) comprenant une extrémité proximale (101) et une extrémité distale (102) opposée comprenant une pointe (103) ; ledit procédé comprenant les étapes de :
- fourniture d'un corps de cathéter (100) comprenant une extrémité proximale (101) et une extrémité distale (102) opposée comprenant une pointe (103) ;
- formation d'une ouverture d'irrigation (111) dans une paroi latérale (110) de ladite pointe (103) ;
- fourniture d'une lumière d'entrée (11) s'étendant de ladite ouverture d'irrigation (111) à ladite extrémité proximale (102) ;
- formation d'une ouverture de drainage (121) dans ladite paroi latérale (110) de ladite pointe (103) ;
- fourniture d'une lumière de sortie (12) s'étendant de ladite ouverture de drainage (121) à ladite extrémité proximale (102) ;
- fourniture d'une lumière de capteur de température (13) comprenant un capteur de température (130), ledit capteur de température (130) comprenant un élément de détection (131) ;
- agencement de ladite lumière de capteur de température (13) plus près de ladite lumière de sortie (12) que de ladite lumière d'entrée (11) dans ledit corps de cathéter (100) ; et
- positionnement dudit élément de détection (131) dans ladite lumière de capteur de température (13) au niveau de ladite pointe (103) de ladite extrémité distale (102) de sorte que ledit élément de détection (131) mesure la température d'un fluide (2) dans une cavité corporelle (10) lorsque ladite extrémité distale (102) dudit corps de cathéter (100) est insérée dans une cavité corporelle (10).
